# EUROPEAN PATENT APPLICATION

(11) **EP 4 523 702 A1**
(43) Date of publication of application: **19.03.2025**
(21) Application number: 23803549.7
(22) Date of filing: 09.05.2023
(51) Int. Cl.: A61K 39/395, A61P 35/00, A61P 35/02, A61P 43/00, C07K 16/28, A61K 47/68, A61K 38/07, A61K 31/4745, A61K 31/537

(54) **COMBINATION OF ANTIBODY-DRUG CONJUGATE AND ANTI-SIRPALPHA ANTIBODY**

(30) Priority: 10.05.2022 JP 2022077725
(71) Applicant: Daiichi Sankyo Company, Limited, Tokyo 103-8426 (JP)
(72) Inventor: KAWASAKI Norihito, Tokyo 103-8426 (JP); ISHIDA Saori, Tokyo 103-8426 (JP); SUE Mayumi, Tokyo 103-8426 (JP); ISHIMOTO Yoko, Tokyo 103-8426 (JP); KAWASE Yumi, Tokyo 103-8426 (JP)
(74) Representative: Marks & Clerk LLP
(86) International application number: PCT/JP2023/017369
(87) International publication number: WO 2023/219072

(57) **Abstract**

Provided are a pharmaceutical composition, treatment method, and anti-SIRPα antibody excellent in antitumor effect and safety aspects. A pharmaceutical composition and treatment method, wherein any one antibody-drug conjugate selected from the group consisting of Sacituzumab govitecan, Polatuzumab vedotin, and Trastuzumab emtansine, and an anti-SIRPα antibody are administered in combination, and an anti-SIRPα antibody to be administered in combination with an antibody-drug conjugate.

## Description

### Technical Field

The present invention relates to a pharmaceutical composition, wherein a specific antibody-drug conjugate and an anti-SIRPα antibody are administered in combination, a therapeutic method comprising administering a specific antibody-drug conjugate and an anti-SIRPα antibody in combination to a subject and/or an anti-SIRPα antibody to be administered in combination with a specific antibody-drug conjugate.

### Background Art

SIRPα (SHPS-1) is a single transmembrane molecule belonging to the Ig super family present in myelocytes such as macrophages, dendritic cells and neutrophils, and glial cells (Non-Patent Literature 1). The extracellular region thereof consists of a single IgV domain and two IgC domains. The IgV domain, which is a connecting position to CD47, is reported to have 10 variants in humans (Non-Patent Literature 2). On the other hand, the intracellular region thereof contains immunoreceptor tyrosine-based inhibition motifs (ITIM). When ITIM binds to CD47, binding to tyrosine dephosphorylation enzymes, SHP-1 and SHP-2, is induced and a suppressive signal is transmitted.

It is reported that SIRPα-CD47 interaction causes a physiological phenomenon, i.e., CD47 on red blood cells binds to SIRPα on macrophages to transmit a "Don't eat me" signal, with the result that unwanted phagocytosis by red blood cells can be avoided (Non-Patent Literature 3). Also, under tumor microenvironments, it is suggested that when CD47, which is highly expressed on tumor cells, binds to SIRPα on macrophages and dendritic cells, phagocytic activity to engulf tumor cells is suppressed. When phagocytic activity is suppressed, the subsequent tumor antigen presentation to T cells and further subsequent tumor immune response will be suppressed. Thus, an immune phenomenon, that is, phagocytosis of tumor cells, is considered as a checkpoint of entry of a tumor antigen.

It has been reported that phagocytic activity to engulf tumor cells is enhanced by inhibiting SIRPα-CD47 interaction by an antibody against CD47, which is a ligand of SIRPα (Non-Patent Literature 4). The same phenomenon as this has been observed when an anti-SIRPα antibody is used under the conditions where an anti-cancer antibody, which has an effecter activity to attract tumor cells to immune cells, is used in combination (Non-Patent Literatures 5 and 6). It is suggested that an anti-CD47 antibody used in an allogeneic mouse cancer-bearing model induces not only an antitumor effect but also tumor immunity (Non-Patent Literature 7). An anti-SIRPα antibody is expected to produce the same effect if it is used in combination with an anti-cancer antibody.

In the meantime, a plurality of antibodies against an immunosuppressive molecule on T cells, such as PD-1/PD-L1, have been developed as an immune checkpoint inhibitor and the clinical effects thereof have been demonstrated (Non-Patent Literatures 8 and 9). SIRPα-CD47 is only one phagocytosis suppressive molecule presently verified. An inhibitory antibody against the molecule is expected to have a possibility of serving as a novel checkpoint inhibitor targeting objects other than T cells and widely exerting an effect on patients resistant to conventional immune checkpoint inhibitors.

Up to present, in studies using an anti-mouse SIRPα antibody (MY-1) and carried out in a human Burkitt's lymphoma subcutaneous transplant model, an antitumor effect has been exerted in a combination use with Rituximab. In a mouse colorectal cancer model, an antitumor effect has been confirmed in a combination use with a PD-1 antibody (Non-Patent Literature 5). In the studies using an anti-mSIRPα antibody of a different clone (P84), an antitumor effect and a life extension effect have been confirmed even in a combination use with an anti-PD-L1 antibody and an anti-4-1BB antibody in a mouse liver cancer model. When the same tumor cells are re-transplanted in mice showing a life extension effect, a further antitumor effect and life extension effect are obtained. This suggests that a strong tumor immune response can be induced by inhibiting different immune checkpoints (Patent Literature 1). The results of these cases show that a combined effect can be obtained by not only a combination use with an anti-cancer antibody having an effector activity, as conventionally expected, but also a combination use with an immune checkpoint inhibitor targeting T cells. The same effect can be expected if an anti-human SIRPα antibody is used.

Recently, patents on an anti-SIRPα antibody have been reported one after another by different companies (Patent Literatures 1, 2 and 3). In Examples of the individual literatures, difference in binding activity to various variants and family molecules (e.g. SIRPβ1, SIRPγ) and difference in antibody IgG subclass are disclosed. For example, OSE-172, which is an IgG4Pro antibody, binds to SIRPα V1 and SIRPβ1 and neither to SIRPα V2 nor SIRPγ; KWAR23, which is an IgG1N279A antibody, binds to all 10 types of SIRPα variants and family molecules, SIRPβ1 and SIRPγ; and ADU-1805, which is an IgG2 antibody, binds to all 10 types of SIRPα variants and SIRPγ. It is still unknown as to which one of the antibodies is the most appropriate as a medicine. Efforts are underway to obtain an excellent antibody.

Further, in studies using an anti-CD47 antibody, it has been reported that not only the combination use with the aforementioned antibody drug but also a combination use with a chemotherapy and/or a radiation therapy widely used as conventional SOC (Standard of Care) exert a sufficient antitumor effect and/or life extension effect. Particularly, in the combination use with a chemotherapy, it was found that when a chemotherapeutic agent is administered in advance, and then an anti-CD47 antibody is administered, a stronger antitumor effect and/or life extension effect are exerted than simultaneous administration of the chemotherapeutic agent and the anti-CD47 antibody (Non-Patent Literature 7). From this, if a chemotherapeutic agent is previously administered to thereby prepare an environment in which a tumor antigen can be easily taken up, take-up of an antigen (immunostimulatory ability) by inhibiting SIRPα-CD47 interaction can be effectively enhanced.

As described above, an anti-SIRPα antibody used in combination with various antitumor agents presumably serves as a drug that can induce a further strong tumor immune response.

An antibody-drug conjugate (ADC) having a drug with cytotoxicity conjugated to an antibody, whose antigen is expressed on the surface of cancer cells and which also binds to an antigen capable of cellular internalization, and therefore can deliver the drug selectively to cancer cells, is thus expected to cause accumulation of the drug within cancer cells and to kill the cancer cells (Non-Patent Literatures 10 to 14).

Sacituzumab govitecan is an antibody-drug conjugate targeting a cancer cell expressing TROP2 and formed of an IgG1-type antibody to which SN-38 is bound as a drug (Non-Patent Literature 15). Polatuzumab vedotin is an antibody-drug conjugate targeting a cancer cell expressing CD79b and formed of an IgG1-type antibody to which monomethyl auristatin E is bound as a drug (Non-Patent Literature 16). Trastuzumab emtansine is an antibody-drug conjugate targeting a cancer cell expressing HER2 and formed of an IgG1-type antibody to which DM1 is bound as a drug (Non-Patent Literature 15). Enfortumab vedotin is an antibody-drug conjugate targeting a cancer cell expressing NECTIN-4 and formed of an IgG1-type antibody to which monomethyl auristatin E is bound as a drug (Non-Patent Literature 15).

However, none of the test results showing a superior combined effect when the foregoing antibody-drug conjugate is used in combination with an anti-SIRPα antibody, or scientific basis for suggesting such a test result, has yet been open to public.

### Citation List

### Patent Literatures

Patent Literature 1: International Publication No. WO 2017/178653
Patent Literature 2: International Publication No. WO 2018/026600
Patent Literature 3: International Publication No. WO 2018/190719
Patent Literature 4: International Publication No. WO 2020/013170

### Non-Patent Literatures

Non-Patent Literature 1: Matozaki et al. Trends in cell biol. 2009 (19) 2, 72-80
Non-Patent Literature 2: Takenaka et al. Nat Immunol. 2007 (8) 12, 1313-1323
Non-Patent Literature 3: Matozaki et al. Trends in cell biol. 2009 (19) 2, 72-80
Non-Patent Literature 4: Liu et al. Plos One, 2015 (10) 9
Non-Patent Literature 5: Yanagita et al. JCI Insight, 2017 (2) 1, 1-15
Non-Patent Literature 6: Ring et al. PNAS, 2017 (114) 49, E10578-E10585
Non-Patent Literature 7: Liu et al, Nat Med. 2015 (21) 10, 1209-1215
Non-Patent Literature 8: Lee et al. The Oncologist, 2017 (22) 11, 1392-1399
Non-Patent Literature 9: Weinstock et al. Clin Can Res. 2017 (23) 16, 4534-4539
Non-Patent Literature 10: Ducry, L., et al., Bioconjugate Chem. (2010) 21, 5-13.
Non-Patent Literature 11: Alley, S. C., et al., Current Opinion in Chemical Biology (2010) 14, 529-537.
Non-Patent Literature 12: Damle N. K. Expert Opin. Biol. Ther. (2004) 4, 1445-1452.
Non-Patent Literature 13: Senter P. D., et al., Nature Biotechnology (2012) 30, 631-637.
Non-Patent Literature 14: Howard A. et al., J Clin Oncol 29: 398-405.
Non-Patent Literature 15: Khongorzul P. et al., Mol. Cancer Res. (2020) 18, 3-19.
Non-Patent Literature 16: Burke JM. et al., Expert Rev. Clin. Pharmacol. (2020) 13, 1073-1083.

### Summary of Invention

### Technical Problem

An antibody-drug conjugate used in the present invention has been confirmed to exert a superior antitumor effect even as a single agent. However, there has been need for obtaining a therapeutic method which can suppress growth of cancer cells in multiple manners and exert a further superior antitumor effect by using the antibody-drug conjugate in combination with another anticancer agent having a different mechanism of action.

An object of the present invention is to provide a pharmaceutical composition, wherein a specific antibody-drug conjugate and an anti-SIRPα antibody are administered in combination, a therapeutic method comprising administering a specific antibody-drug conjugate and an anti-SIRPα antibody in combination to a subject, and/or an anti-SIRPα antibody to be administered in combination with a specific antibody-drug conjugate.

### Solution to Problem

As a result of diligent studies in order to solve the above problems, the present inventors have found that combined administration of a specific antibody-drug conjugate and an anti-SIRPα antibody exhibits a superior combined effect, and completed the present invention.

That is, the present invention provides the following [1] to [21]:
[1] A pharmaceutical composition, wherein an antibody-drug conjugate and an anti-SIRPα antibody are administered in combination, and
   the antibody-drug conjugate is any one antibody-drug conjugate selected from the group consisting of Sacituzumab govitecan, Polatuzumab vedotin, and Trastuzumab emtansine.
[2] The pharmaceutical composition according to [1], wherein the antibody-drug conjugate is Sacituzumab govitecan.
[3] The pharmaceutical composition according to [1], wherein the antibody-drug conjugate is Polatuzumab vedotin.
[4] The pharmaceutical composition according to [1], wherein the antibody-drug conjugate is Trastuzumab emtansine.
[5] The pharmaceutical composition according to any one of [1] to [4], wherein the anti-SIRPα antibody is any one of the following antibodies (1) to (5):
   (1) an antibody comprising a heavy chain consisting of an amino acid sequence consisting of amino acid residues 20 to 466 of SEQ ID NO: 1 and a light chain consisting of an amino acid sequence consisting of amino acid residues 21 to 234 of SEQ ID NO: 4;
   (2) an antibody comprising the heavy chain consisting of the amino acid sequence consisting of amino acid residues 20 to 466 of SEQ ID NO: 1 and a light chain consisting of an amino acid sequence consisting of amino acid residues 21 to 234 of SEQ ID NO: 5;
   (3) an antibody comprising a heavy chain consisting of an amino acid sequence consisting of amino acid residues 20 to 466 of SEQ ID NO: 2 and a light chain consisting of an amino acid sequence consisting of amino acid residues 21 to 234 of SEQ ID NO: 3;
   (4) an antibody comprising the heavy chain consisting of the amino acid sequence consisting of amino acid residues 20 to 466 of SEQ ID NO: 2 and a light chain consisting of an amino acid sequence consisting of amino acid residues 21 to 234 of SEQ ID NO: 4; and
   (5) an antibody according to any one of (1) to (4), lacking a lysine residue at the carboxyl terminus of the heavy chain.
[6] The pharmaceutical composition according to any one of [1] to [5], wherein the antibody-drug conjugate and the anti-SIRPα antibody are separately contained as active components in different formulations, and are administered simultaneously or at different times.
[7] The pharmaceutical composition according to any one of [1] to [6], wherein the composition is for treating at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[8] A therapeutic method comprising administering an antibody-drug conjugate and an anti-SIRPα antibody in combination to a subject in need of treatment, wherein the antibody-drug conjugate is any one antibody-drug conjugate selected from the group consisting of Sacituzumab govitecan, Polatuzumab vedotin, and Trastuzumab emtansine.
[9] The therapeutic method according to [8], wherein the antibody-drug conjugate is Sacituzumab govitecan.
[10] The therapeutic method according to [8], wherein the antibody-drug conjugate is Polatuzumab vedotin.
[11] The therapeutic method according to [8], wherein the antibody-drug conjugate is Trastuzumab emtansine.
[12] The therapeutic method according to any one of [8] to [11], wherein the anti-SIRPα antibody is any one of the following antibodies (1) to (5):
   (1) an antibody comprising a heavy chain consisting of an amino acid sequence consisting of amino acid residues 20 to 466 of SEQ ID NO: 1 and a light chain consisting of an amino acid sequence consisting of amino acid residues 21 to 234 of SEQ ID NO: 4;
   (2) an antibody comprising the heavy chain consisting of the amino acid sequence consisting of amino acid residues 20 to 466 of SEQ ID NO: 1 and a light chain consisting of an amino acid sequence consisting of amino acid residues 21 to 234 of SEQ ID NO: 5;
   (3) an antibody comprising a heavy chain consisting of an amino acid sequence consisting of amino acid residues 20 to 466 of SEQ ID NO: 2 and a light chain consisting of an amino acid sequence consisting of amino acid residues 21 to 234 of SEQ ID NO: 3;
   (4) an antibody comprising the heavy chain consisting of the amino acid sequence consisting of amino acid residues 20 to 466 of SEQ ID NO: 2 and a light chain consisting of an amino acid sequence consisting of amino acid residues 21 to 234 of SEQ ID NO: 4; and
   (5) an antibody according to any one of (1) to (4), lacking a lysine residue at the carboxyl terminus of the heavy chain.
[13] The therapeutic method according to any one of [8] to [12], wherein the antibody-drug conjugate and the anti-SIRPα antibody are separately contained as active components in different formulations, and are administered simultaneously or at different times.
[14] The therapeutic method according to any one of [8] to [13], wherein the method is for treating at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[15] An anti-SIRPα antibody to be administered in combination with an antibody-drug conjugate, wherein the antibody-drug conjugate is any one antibody-drug conjugate selected from the group consisting of Sacituzumab govitecan, Polatuzumab vedotin, and Trastuzumab emtansine.
[16] The anti-SIRPα antibody according to [15], wherein the antibody-drug conjugate is Sacituzumab govitecan.
[17] The anti-SIRPα antibody according to [15], wherein the antibody-drug conjugate is Polatuzumab vedotin.
[18] The anti-SIRPα antibody according to [15], wherein the antibody-drug conjugate is Trastuzumab emtansine.
[19] The anti-SIRPα antibody according to any one of [15] to [18], wherein the anti-SIRPα antibody is any one of the following antibodies (1) to (5):
   (1) an antibody comprising a heavy chain consisting of an amino acid sequence consisting of amino acid residues 20 to 466 of SEQ ID NO: 1 and a light chain consisting of an amino acid sequence consisting of amino acid residues 21 to 234 of SEQ ID NO: 4;
   (2) an antibody comprising the heavy chain consisting of the amino acid sequence consisting of amino acid residues 20 to 466 of SEQ ID NO: 1 and a light chain consisting of an amino acid sequence consisting of amino acid residues 21 to 234 of SEQ ID NO: 5;
   (3) an antibody comprising a heavy chain consisting of an amino acid sequence consisting of amino acid residues 20 to 466 of SEQ ID NO: 2 and a light chain consisting of an amino acid sequence consisting of amino acid residues 21 to 234 of SEQ ID NO: 3;
   (4) an antibody comprising the heavy chain consisting of the amino acid sequence consisting of amino acid residues 20 to 466 of SEQ ID NO: 2 and a light chain consisting of an amino acid sequence consisting of amino acid residues 21 to 234 of SEQ ID NO: 4; and
   (5) an antibody according to any one of (1) to (4), lacking a lysine residue at the carboxyl terminus of the heavy chain.
[20] The anti-SIRPα antibody according to any one of [15] to [19], wherein the antibody-drug conjugate and the anti-SIRPα antibody are separately contained as active components in different formulations, and are administered simultaneously or at different times.
[21] The anti-SIRPα antibody according to any one of [15] to [20], wherein the antibody is for treating at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

Furthermore, the present invention provides the following [22] and [23].
[22] A method for using an anti-SIRPα antibody for producing a pharmaceutical product for treating cancer, wherein the anti-SIRPα antibody is administered in combination with an antibody-drug conjugate, and the antibody-drug conjugate is any one antibody-drug conjugate selected from the group consisting of Sacituzumab govitecan, Polatuzumab vedotin, and Trastuzumab emtansine.
[23] A method for using an anti-SIRPα antibody for treating cancer wherein the anti-SIRPα antibody is administered in combination with an antibody-drug conjugate, and the antibody-drug conjugate is any one antibody-drug conjugate selected from the group consisting of Sacituzumab govitecan, Polatuzumab vedotin, and Trastuzumab emtansine.

### Advantageous Effects of Invention

The present invention can provide a pharmaceutical composition, wherein a specific antibody-drug conjugate and an anti-SIRPα antibody are administered in combination, a therapeutic method comprising administering a specific antibody-drug conjugate and an anti-SIRPα antibody in combination to a subject, and/or an anti-SIRPα antibody to be administered in combination with a specific antibody-drug conjugate.

### Brief Description of Drawings

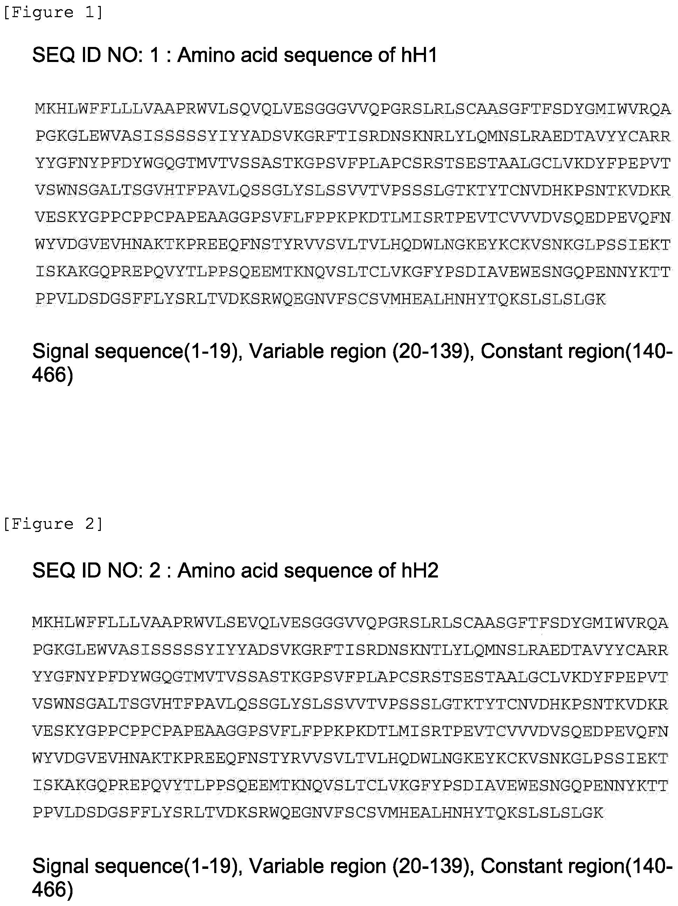

[Figure 1] Figure 1 is a diagram showing an amino acid sequence of hH1 heavy chain of humanized anti-SIRPα antibody D13 (SEQ ID NO: 1).
[Figure 2] Figure 2 is a diagram showing an amino acid sequence of hH2 heavy chain of humanized anti-SIRPα antibody D13 (SEQ ID NO: 2).

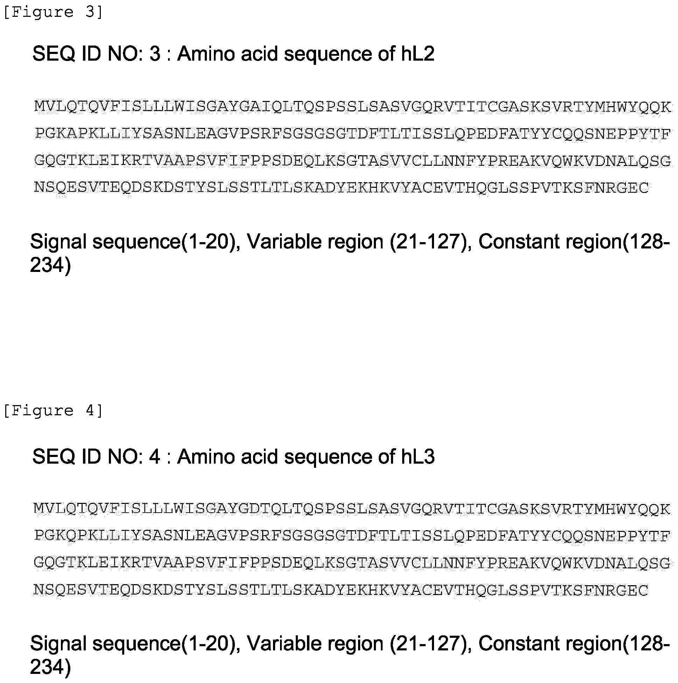

[Figure 3] Figure 3 is a diagram showing an amino acid sequence of hL2 light chain of humanized anti-SIRPα antibody D13 (SEQ ID NO: 3).
[Figure 4] Figure 4 is a diagram showing an amino acid sequence of hL3 light chain of humanized anti-SIRPα antibody D13 (SEQ ID NO: 4).
[Figure 5] Figure 5 is a diagram showing an amino acid sequence of hL4 light chain of humanized anti-SIRPα antibody D13 (SEQ ID NO: 5).
[Figure 6] Figure 6 is a diagram showing CDR sequences of anti-SIRPα antibody cD13 (SEQ ID NO: 6 to 11).

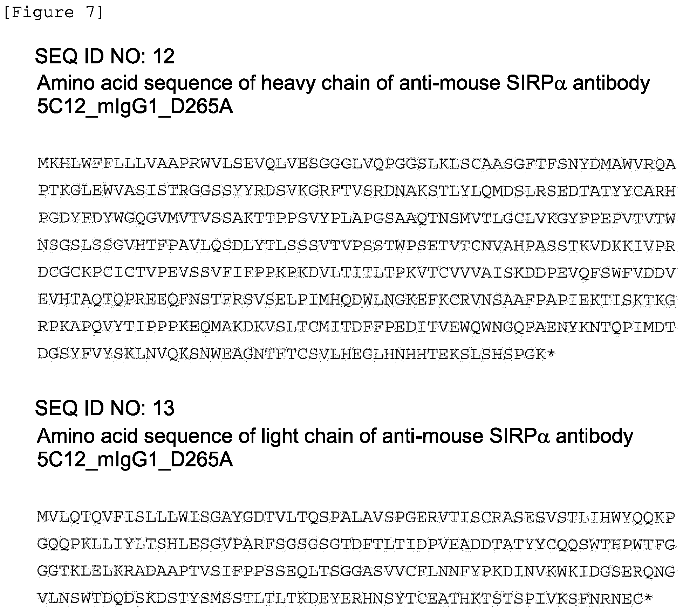

[Figure 7] Figure 7 is a diagram showing an amino acid sequence of a heavy chain of a 5C12 anti-mouse SIRPα antibody (SEQ ID NO: 12) and an amino acid sequence of a light chain thereof (SEQ ID NO: 13).
[Figure 8] Figure 8 is a graph showing ADCP activity of an anti-SIRPα antibody and/or Sacituzumab govitecan to TROP2-positive human gastric cancer cells.
[Figure 9] Figure 9 is a graph showing ADCP activity of an anti-SIRPα antibody and/or Polatuzumab vedotin to CD79b-positive human Burkitt's lymphoma cells.
[Figure 10] Figure 10 is a graph showing ADCP activity of an anti-SIRPα antibody and/or Trastuzumab emtansine to HER2-positive human breast cancer cells.
[Figure 11-1] (Figure 11A) Figure 11A shows the overview of an antitumor study by an anti-SIRPα antibody, and/or Sacituzumab govitecan in a mouse in which TROP2 expressing mouse colorectal cancer cells have been transplanted. (Figure 11B) Figure 11B is a graph showing antitumor effects by an anti-SIRPα antibody and/or Sacituzumab govitecan in a mouse in which TROP2 expressing mouse colorectal cancer cells have been transplanted.
[Figure 11-2] (Figure 11C) Figure 11C is a graph showing an antitumor effect by an anti-SIRPα antibody and/or Sacituzumab govitecan in individual mice in which TROP2 expressing mouse colorectal cancer cells have been transplanted.
[Figure 12-1] (Figure 12A) Figure 12A shows the overview of an antitumor study by anti-SIRPα antibody, and/or Trastuzumab emtansine in a mouse in which HER2-expressing mouse colorectal cancer cells have been transplanted.
(Figure 12B) Figure 12B is a graph showing an antitumor effect by an anti-SIRPα antibody and/or Trastuzumab emtansine in a mouse in which HER2-expressing mouse colorectal cancer cells have been transplanted.

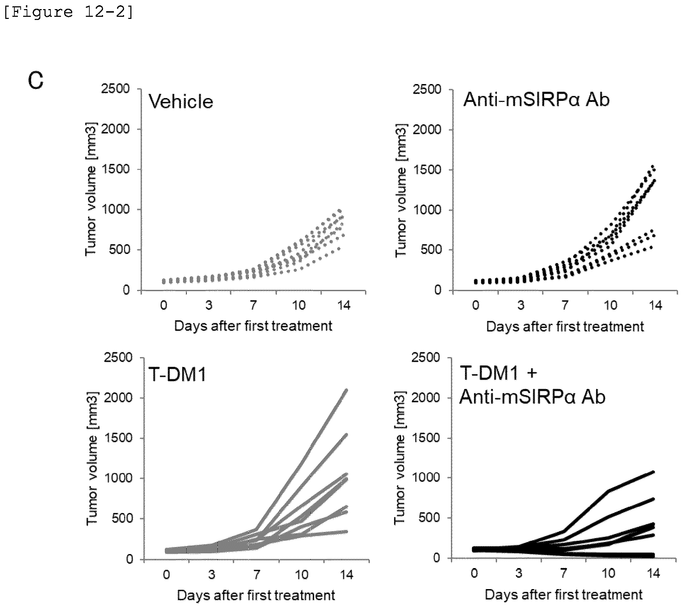

[Figure 12-2] (Figure 12C) Figure 12C is a graph showing an antitumor effect by an anti-SIRPα antibody and/or Trastuzumab emtansine in mouse individuals in which HER2-expressing mouse colorectal cancer cells have been transplanted.

### Description of Embodiments

Hereinafter, preferred modes for carrying out the present invention are described. The embodiments described below are given merely for illustrating one example of a typical embodiment of the present invention and are not intended to limit the scope of the present invention.

### 1. Antibody-drug conjugate

The antibody-drug conjugate used in the present invention is Sacituzumab govitecan, Polatuzumab vedotin, Trastuzumab emtansine, or Enfortumab vedotin.

Sacituzumab govitecan is an antibody-drug conjugate targeting a cancer cell expressing TROP2 and formed of an IgG1-type antibody to which SN-38 is bound as a drug (Non-Patent Literature 15). Polatuzumab vedotin is an antibody-drug conjugate targeting a cancer cell expressing CD79b and formed of an IgG1-type antibody to which monomethyl auristatin E is bound as a drug (Non-Patent Literature 16). Trastuzumab emtansine is an antibody-drug conjugate targeting a cancer cell expressing HER2 and formed of an IgG1-type antibody to which DM1 is bound as a drug (Non-Patent Literature 15). Enfortumab vedotin is an antibody-drug conjugate targeting a cancer cell expressing NECTIN-4 and formed of an IgG1-type antibody to which monomethyl auristatin E is bound as a drug (Non-Patent Literature 15).

### 2. Anti-SIRPα antibody

SIRPα (signal regulatory protein α) is a single transmembrane molecule belonging to the Ig super family present in myelocytes such as macrophages, dendritic cells and neutrophils, and glial cells. The extracellular region thereof consists of a single IgV domain and two IgC domains. The IgV domain, in which a connecting position to CD47 is present, is reported to have 10 variants V1 to V10 in humans. The extracellular IgV domain of the SIRPα protein is one of the three extracellular Ig-like domains constituting SIRPα protein. Of the domains, V1 and V2 are major variants. The anti-SIRPα antibody of the present invention binds to all variants including the major variants V1 and V2. In the present invention, "SIRPα" will be sometimes referred to as "SIRPA".

The amino acid sequence of a human SIRPα protein is disclosed in GenBank Accession No.: NP_001035111.

The anti-SIRPα antibody used in the present invention can be obtained in the same manner as described in International Publication No. WO 2020/013170.

The monoclonal antibody used in the present invention can be obtained by immunizing a mammal such as a mouse, a rat, a rabbit, a hamster, a guinea pig, a horse, a monkey, a dog, a pig, a cow, a goat, a sheep, with SIRPα or a fragment thereof used as an immunogen, fusing the spleen cells and myeloma cells to obtain hybridoma and allowing the hybridoma to produce and secrete the antibody. The hybridoma can be produced by a method known in the art.

SIRPα serving as immunogen can be chemically synthesized based on sequence information or can be obtained as a recombinant protein, which is produced based on a DNA sequence encoding a protein and in accordance with a method known in the art.

The antibody can be screened in any method; preferably, by Cell-ELISA using animal cells transfected with DNA encoding SIRPα.

The anti-SIRPα antibody used in the present invention inhibits binding between SIRPα and CD47.

Tumor cells highly express CD47. When SIRPα expressed on phagocytes having phagocytic activity binds to CD47 and they interact, a "Don't-eat-me" signal is transmitted to the phagocytes. In this way, the tumor cells escape from phagocytosis by the phagocytes. The anti-SIRPα antibody inhibits the binding between SIRPα and CD47, thereby inhibiting transmission of a "Don'teat-me" signal from tumor cells to phagocytes, thereby enhancing phagocytosis by phagocytes to engulf tumor cells. As a result, an antitumor effect can be exerted. Examples of the phagocytes having phagocytic activity include macrophages such as M1 and M2 macrophages and dendritic cells such as immature dendritic cells (imDC).

In this case, when an anti-SIRPα antibody having an effector function binds to an Fc receptor such as Fcy receptor of effector cells such as phagocytes (e.g., macrophages), natural killer cells and T cells, the anti-SIRPα antibody attacks autologous effector cells such as PBMC (peripheral blood mononuclear cells) and macrophages due to ADCC (Antibody Dependent Cellular Cytotoxicity) and ADCP (Antibody Dependent Cellular Phagocytosis).

To avoid attacking against autologous cells, the anti-SIRPα antibody used in the present invention is reduced in effector function. As a result, the anti-SIRPα antibody used in the present invention only has a function to suppress the binding between SIRPα and CD47 and never binds to an Fc receptor of effector cells; that is, an effector function is not produced.

The anti-SIRPα antibody used in the present invention, since it does not attack autologous immune cells, can be safely used as a medicine having no side effects.

However, the anti-SIRPα antibody used in the present invention, since it is reduced in effector function, does not produce a sufficient antitumor effect if it is used alone. Thus, the anti-SIRPα antibody is used in combination with another antitumor agent.

To reduce an effector function, it is necessary for an Fc part of an anti-SIRPα antibody not to bind to Fc receptors of macrophages and T cells. For the reason, a subclass of the anti-SIRPα antibody used in the present invention is replaced with that derived from IgG4. Generally, of human IgG subclasses, IgG4 is known as a subclass low in effector function such as ADCC activity, CDC activity and/or ADCP activity (Bruggemann et al., J. Exp. Med., 1351-1361, 1987). IgG4 is used as one of IgG formats for avoiding toxicity due to cell damage via effector function, when a therapeutic antibody is used for targeting a molecule expressed in a healthy organ (e.g. Opdivo). Note that the effector function of IgG4 subclass is low, but not zero. Then, in the anti-SIRPα antibody used in the present invention, a mutation such as a mutation that can further reduce the effector function, more specifically, a mutation such as a substitution with at least one amino acid for reducing ADCC and/or ADCP activity, is introduced into the heavy chain constant region. Examples of such a mutation include a substitution of phenylalanine with alanine at position 234 (F234A) according to EU index by Kabat et al. (Kabat et. al., Sequences of proteins of immunological interest, 1991 Fifth edition), and a substitution of leucine with alanine at position 235 (L235A) (Parekh et al., mAbs, 310-318, 2012). Such a mutation of an antibody refers to FALA mutation.

In IgG4, the S-S bond between antibody heavy chains is not stably formed. In order to improve the stability, a mutation for promoting the S-S bond between antibody heavy chains is introduced. Examples of the mutation include a substitution of serine with proline at position 228 (S228P) according to EU index by Kabat et al. (ANGAL et.al., Molecular Immunology, 105-108, 1993). The mutation of an antibody is referred to as PRO mutation.

In the constant region of the anti-SIRPα antibody used in the present invention, the aforementioned FALA mutation and PRO mutation may be simultaneously introduced (Vafa et.al., Methods, 65, 114-126, 2014). An IgG4 heavy chain having both the FALA mutation and Pro mutation is referred to as "IgG4proFALA" heavy chain, "IgG4PFALA" heavy chain, or "IgG4pf" heavy chain.

Of the human IgG subclasses, human IgG1 has a very strong effector function such as CDC activity via complement binding and an antibody-dependent cellular cytotoxity activity (Bruggemann et al., J. Exp. Med., 1351-1361, 1987), and is used as an IgG format exerting a therapeutic effect by promoting cell death of cancer cells due to cell damage via effector function when a therapeutic antibody is used for targeting a molecule highly expressed in cancer (e.g. Trastuzumab, Rituximab). When IgG1 is used as an isotype of the anti-SIRPα antibody used in the present invention, an effector function can be controlled by substituting part of the amino acid residues of the constant region (see, WO 88/007089, WO 94/28027, WO 94/29351). Examples of IgG1 mutant attenuated in effector function include IgG1 LALA (IgG1-L234A, L235A) and IgG1 LAGA (IgG1-L235A, G237A). As the constant region of the anti-SIRPα antibody used in the present invention, the IgG1 heavy-chain constant region having these mutations introduced therein can be used.

Of the human IgG subclasses, human IgG2 has a very weak effector function such as a CDC activity via complement binding and an antibody-dependent cellular cytotoxity activity (Bruggemann et al., J. Exp. Med., 1351-1361, 1987), and is used as one of the IgG formats for avoiding toxicity due to cell damage via effector function when a therapeutic antibody is used for targeting a molecule expressed in a healthy organ (e.g. Denosumab, Evolocumab, Brodalumab). As the constant region of the anti-SIRPα antibody used in the present invention, the IgG2 heavy-chain constant region can be used.

In the anti-SIRPα antibody used in the present invention, modified variants of the antibody are also included. The modified variant refers to a variant obtained by subjecting the anti-SIRPα antibody to be used in the present invention to chemical or biological modification. Examples of the chemically modified variant include variants having a linkage of a chemical moiety to an amino acid skeleton or variants having a linkage of a chemical moiety to an N-linked or O-linked carbohydrate chain. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N-terminus by expression using a prokaryotic host cell. Further, an antibody labeled so as to enable the detection or isolation of the anti-SIRPα antibody or antigen used in the present invention, for example, an enzyme-labeled antibody, a fluorescence-labeled antibody and an affinity-labeled antibody, are also included in the meaning of the modified variant. Such a modified variant of the anti-SIRPα antibody used in the present invention is useful for, e.g., improving the stability and blood retention of the antibody, reducing the antigenicity thereof, detecting or isolating an antibody or an antigen.

Note that, it is known that a lysine residue at the carboxyl terminus of the heavy chain of an antibody produced in a cultured mammalian cell is deleted (Journal of Chromatography A, 705: 129-134 (1995)). It is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (e.g., activation of complement, antibody-dependent cellular cytotoxicity,) of the antibody. Therefore, in the anti-SIRPα antibody used in the present invention, antibodies subjected to such modification and functional fragments thereof are also included, and deletion variants in which one or two amino acids have been deleted at the carboxyl terminus of the heavy chain, deletion variants having an amidated residue (for example, a heavy chain in which the carboxyl terminal proline residue has been amidated) are also included. Note that, the type of deletion variant having a deletion at the carboxyl terminus of the heavy chain of the anti-SIRPα antibody used in the present invention is not limited to the above variants as long as the antigen-binding affinity and the effector function are conserved. The two heavy chains constituting the anti-SIRPα antibody used in the present invention may be one selected from the group consisting of a full-length heavy chain and the above-described heavy chain having a deletion, or may be of two types in combination selected therefrom. The ratio of the amount of each deletion variant can be affected by the type of cultured mammalian cells which produce anti-SIRPα antibody used in the present invention and the culture conditions; however, an antibody in which one amino acid residue at the carboxyl terminus has been deleted in both of the two heavy chains in the anti-SIRPα antibody used in the present invention can be preferably exemplified.

The anti-SIRPα antibody used in the present invention includes a chimeric antibody modified in order to decline heterogeneous antigenicity to humans and a humanized antibody. The humanized antibody is also referred to as a CDR transplanted antibody.

The chimeric antibody refers to an antibody consisting of a light chain variable region and a heavy chain variable region of an antibody of a non-human animal, and a light chain constant region and a heavy chain constant region of a human antibody. The chimeric antibody can be prepared by taking cDNA encoding a light chain variable region and cDNA encoding a heavy chain variable region from a hybridoma producing an anti-SIRPα antibody, and inserting the cDNAs into an expression vector having cDNA encoding a light chain constant region and a heavy chain constant region of a human antibody to construct a chimeric antibody expression vector, introducing the chimeric antibody expression vector into host cells and allowing expression of the antibody.

The heavy chain constant region is formed of three domains C_{H}1, C_{H}2 and C_{H}3. In the anti-SIRPα antibody used in the present invention, human heavy-chain constant region of the chimeric antibody is an IgG4-subclass heavy-chain constant region having Pro mutation and FALA mutation, that is, a heavy chain constant region IgG4proFALA. The light chain constant region may be sufficient if it belongs to human Ig, and is κ or λ constant region.

Examples of the chimeric antibody of the anti-SIRPα antibody used in the present invention include a chimeric antibody having a variable region of rat anti-human SIRPα monoclonal antibody D13, i.e. antibody cD13. Antibody cD13 is an antibody having a high binding activity to bind to human SIRPα and having a high inhibitory activity against the binding between SIRPα and CD47.

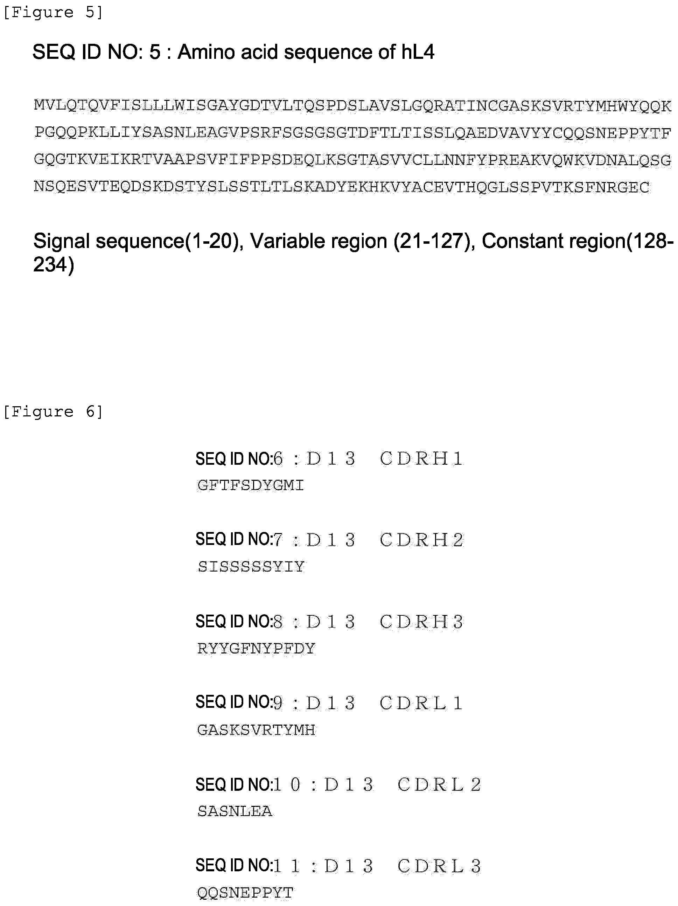

Antibody cD13 comprises, as CDRs (complementarity determining regions) of a light chain variable region, CDRL1 consisting of an amino acid sequence (GASKSVRTYMH) represented by SEQ ID NO: 9, CDRL2 consisting of an amino acid sequence (SASNLEA) represented by SEQ ID NO: 10, and CDRL3 consisting of an amino acid sequence (QQSNEPPYT) represented by SEQ ID NO: 11, and further, as CDRs of a heavy chain variable region, CDRH1 consisting of an amino acid sequence (GFTFSDYGMI) represented by SEQ ID NO: 6, and CDRH2 consisting of an amino acid sequence (SISSSSSYIY) represented by SEQ ID NO: 7, and CDRH3 consisting of an amino acid sequence (RYYGFNYPFDY) represented by SEQ ID NO: 8 (Figure 6).

More specifically, the anti-SIRPα antibody used in the present invention is an antibody comprising CDRL1 consisting of the amino acid sequence represented by SEQ ID NO: 9, CDRL2 consisting of the amino acid sequence represented by SEQ ID NO: 10, and CDRL3 consisting of the amino acid sequence represented by SEQ ID NO: 11, and, as CDRs of the heavy chain variable region, CDRH1 consisting of the amino acid sequence represented by SEQ ID NO: 6, CDRH2 consisting of the amino acid sequence represented by SEQ ID NO: 7, and CDRH3 consisting of the amino acid sequence represented by SEQ ID NO: 8.

The aforementioned CDRs include CDRs consisting of amino acid sequences having deletion, substitution and/or addition of one or several amino acids, preferably one or two amino acids, and further preferably, a single amino acid in each of the amino acid sequences of the CDRs.

A humanized antibody (CDR-transplanted antibody) refers to an antibody prepared by transplanting amino acid sequences of CDRs of a light chain variable region and a heavy chain variable region of an antibody of a non-human animal to appropriate positions of a light chain variable region and a heavy chain variable region of a human antibody.

The humanized anti-SIRPα antibody of the present invention can be produced by constructing cDNA encoding variable regions, which are obtained by transplanting the amino acid sequences of CDRs of a light chain variable region and a heavy chain variable region of an antibody of a non-human animal, which is produced from a hybridoma producing a monoclonal antibody binding to human SIRPα to inhibit the binding between SIRPα and CD47, thereby enhancing macrophage phagocytosis, to framework (FR) regions of a light chain variable region and a heavy chain variable region of a human antibody; inserting the cDNA into an animal-cell expression vector having genes encoding a light chain constant region and a heavy chain constant region of a human antibody to construct a humanized antibody expression vector; introducing the vector into animal cells; and expressing the cDNA.

More specifically, a DNA sequence designed such that the CDRs of an antibody cD13 are linked to framework regions of a human antibody, may be synthesized. The human antibody framework regions to be linked via CDRs are selected such that CDRs form satisfactory antigen connecting positions. If necessary, an amino acid of the framework region in the variable region of the antibody may be substituted such that CDRs of the humanized antibody form an appropriate antigen connecting position. A humanized antibody can be prepared by transplanting CDRs in accordance with CDR grafting technology known in the art.

As the heavy chain of the humanized antibody having CDRs (6 CDRs formed of amino acids represented by SEQ ID NOs: 6 to 11) of heavy chain and light chain variable regions of antibody cD13, which is prepared in accordance with the aforementioned method and has substitution of part of the amino acids in framework regions of the variable region, humanized antibody heavy chain hH1 and humanized antibody heavy chain hH2 can be exemplified. As the light chain of the humanized antibody having CDRs of a light chain variable region of antibody D13 and having substitution of part of the amino acids in framework regions of the variable region, humanized antibody light chain hL2, humanized antibody light chain hL3 and humanized antibody light chain hL4 can be exemplified.

The full-length amino acid sequence of humanized antibody heavy chain hH1 is represented by SEQ ID NO: 1. The full-length amino acid sequence of humanized antibody heavy chain hH2 is represented by SEQ ID NO: 2. In SEQ ID NOs: 1 and 2, the amino acid sequence consisting of 1st to 19th amino acid residues is an amino acid sequence of a signal sequence; the amino acid sequence consisting of 20 to 139th amino acid residues is an amino acid sequence of a variable region; and the amino acid sequence consisting of 140 to 466th amino acid residues is an amino acid sequence of a constant region.

The anti-SIRPα antibody used in the present invention includes an antibody having a heavy chain variable region consisting of 20 to 139th amino acid residues of SEQ ID NO: 1 or 2 and a heavy chain constant region consisting of 140 to 466th amino acid residues thereof.

The full-length amino acid sequence of humanized antibody light chain hL2 is represented by SEQ ID NO: 3. The full-length amino acid sequence of humanized antibody light chain hL3 is represented by SEQ ID NO: 4. The full-length amino acid sequence of humanized antibody light chain hL4 is represented by SEQ ID NO: 5. In SEQ ID NOs: 3, 4 and 5, the amino acid sequence consisting of 1 to 20th amino acid residues is an amino acid sequence of a signal sequence; the amino acid sequence consisting of 21 to 127th amino acid residues is an amino acid sequence of a variable region; and the amino acid sequence consisting of 128 to 234th amino acid residues is an amino acid sequence of a constant region.

The anti-SIRPα antibody used in the present invention includes antibodies having light chain variable regions consisting of 21 to 127th amino acid residues of SEQ ID NO: 3, 4 and 5 and light chain constant regions consisting of 128 to 234th amino acid residues thereof.

The heavy chain constant region of a humanized antibody is a heavy chain constant region of IgG4 subclass, more specifically, heavy chain constant region IgG4proFALA having a Pro mutation and a FALA mutation.

Examples of the antibody having high binding activity to human SIRPα and a high inhibitory activity against the binding between SIRPα and CD47 include an antibody consisting of humanized antibody heavy chain hH1 and humanized antibody light chain hL3 (hD13_H1L3 antibody); an antibody consisting of humanized antibody heavy chain hH1 and humanized antibody light chain hL4 (hD13_H1L4 antibody); an antibody consisting of humanized antibody heavy chain hH2 and humanized antibody light chain hL2 (hD13_H2L2 antibody); and an antibody consisting of humanized antibody heavy chain hH2 and humanized antibody light chain hL3 (hD13_H2L3 antibody).

The hD13_H1L3 antibody is an antibody having a heavy chain consisting of 20 to 466th amino acid residues of SEQ ID NO: 1 and a light chain consisting of 21 to 234th amino acid residues of SEQ ID NO: 4.

The hD13_H1L4 antibody is an antibody having a heavy chain consisting of 20 to 466th amino acid residues of SEQ ID NO: 1 and a light chain consisting of 21 to 234th amino acid residues of SEQ ID NO: 5.

The hD13_H2L2 antibody is an antibody having a heavy chain consisting of 20 to 466th amino acid residues of SEQ ID NO: 2 and a light chain consisting of 21 to 234th amino acid residues of SEQ ID NO: 3.

The hD13_H2L3 antibody is an antibody having a heavy chain consisting of 20 to 466th amino acid residues of SEQ ID NO: 2 and a light chain consisting of 21 to 234th amino acid residues of SEQ ID NO: 4.

Note that, in an antibody produced in a cultured mammalian cell, it is known that a lysine residue at the carboxyl terminus of the heavy chain is deleted (Tsubaki et al., Int. J. Biol. Macromol, 139-147, 2013). However, the deletion of the heavy chain sequence does not affect the antigen-binding affinity and the effector function (e.g. activation of a complement and antibody-dependent cellular cytotoxicity) of the antibody. Thus, in the present invention, an antibody lacking a lysine residue at the carboxyl terminus of the heavy chain is included.

The cancer therapeutic agent used in the present invention can contain a therapeutically effective amount of an anti-SIRPα antibody and a pharmaceutically acceptable carrier, diluent, solubilizer, emulsifier, preservative, auxiliary agent and others. The "pharmaceutically acceptable carrier" and others can be appropriately selected from a wide range in accordance with the type of target disease and the dosage form of a drug. A method for administering an antitumor agent according to the present invention can be appropriately selected, for example, administration by injection can be selected. Examples of the injection that can be employed include local injection, intraperitoneal injection, selective intravenous injection, intravenous injection, subcutaneous injection and organ-perfusate injection. The injection solution can be formulated by using a carrier consisting of a salt solution, a glucose solution, or a mixture of a salt solution and a glucose solution, and any one of buffers. Alternatively, the injection solution may be prepared by formulating a powder preparation and mixing the powder preparation with the aforementioned liquid carrier, when used.

Other administration methods can be appropriately selected together with development of formulations. For example, for oral administration, oral liquids, powders, pills, capsules and tablets can be applied. In the case of an oral liquid, an oral liquid preparation such as a suspension and a syrup can be produced by using water; a sugar such as sucrose, sorbitol and fructose; a glycol such as polyethylene glycol; an oil such as sesame oil and soybean oil; a preservative such as alkyl parahydroxybenzoate; and a flavor such as strawberry flavor and peppermint flavor. Powders, pills, capsules and tablets can be formulated by using, e.g., an excipient such as lactose, glucose, sucrose and mannitol; a disintegrant such as starch and soda alginate; a lubricant such as magnesium stearate and talc; a binder such as polyvinyl alcohol, hydroxypropyl cellulose and gelatin; a surfactant such as a fatty acid ester; and a plasticizer such as glycerin. Tablets and capsules are easily administered. In this respect, they are preferable unit dosage forms of the composition of the invention. In producing tablets and capsules, a solid production carrier is used.

### 3. Medicine

Now, a pharmaceutical composition and a therapeutic method according to the present invention, wherein an antibody-drug conjugate and an anti-SIRPα antibody are administered in combination, will be described.

The pharmaceutical composition and therapeutic method of the present invention may be those in which the antibody-drug conjugate and the anti-SIRPα antibody are separately contained as active components in different formulations and are administered simultaneously or at different times, or may be those in which the antibody-drug conjugate and the anti-SIRPα antibody are contained as active components in a single formulation and administered.

The pharmaceutical composition and therapeutic method of the present invention can be used for treating cancer, preferably for treating at least one disease selected from the group consisting of breast cancer, gastric cancer (also called to as gastric adenocarcinoma), colorectal cancer (also called colon and rectal cancer, and including colon cancer and rectal cancer), lung cancer (including small cell lung cancer and non-small cell lung cancer), esophageal cancer, head-and-neck cancer (including salivary gland cancer and pharyngeal cancer), esophagogastric junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, and melanoma; more preferably for treating at least one cancer selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, and uterine carcinosarcoma; and even more preferably for treating at least one cancer selected from the group consisting of breast cancer, gastric cancer, lung cancer, and ovarian cancer.

The kinds of antibodies that would be involved in a preferable antibody-drug conjugate among the antibody-drug conjugates used in the present invention can be determined by examining the type of cancer and tumor markers. For example, if TROP2 expression is found in cancer, Sacituzumab govitecan can preferably be used; if CD79b expression is found in cancer, Polatuzumab vedotin can preferably be used; if HER2 expression is found in cancer, Trastuzumab emtansine can preferably be used; and if NECTIN-4 expression is found in cancer, Enfortumab vedotin can preferably be used.

The presence or absence of TROP2, CD79b, HER2, and NECTIN-4 can be checked by, for example, collecting tumor tissue from a cancer patient, and subjecting the formalin-fixed paraffin-embedded specimen (FFPE) to an examination at a gene product (protein) level such as an immunohistochemistry (IHC) method, a flow cytometry, a western blot method, or examination at a gene translation level such as an in situ hybridization method (ISH), a quantitative PCR method (q-PCR), a microarray analysis, or alternatively, can also be checked by collecting cell-free blood circulating tumor DNA (ctDNA) from a cancer patient and subjecting to an examination which uses a method such as next generation sequencing (NGS).

The pharmaceutical composition and therapeutic method of the present invention can preferably be used for mammals, and can more preferably be used for human.

An antitumor effect of the pharmaceutical composition and therapeutic method of the present invention can be confirmed by, for example, generating a model in which cancer cells are transplanted to a test animal, and measuring reduction of tumor volume, life-prolonging effect due to treating the pharmaceutical composition and therapeutic method of the present invention. Furthermore, comparison to antitumor effects in single administrations of each of the antibody-drug conjugate and the anti-SIRPα antibody used in the present invention can provide confirmation of a combined effect of the antibody-drug conjugate and the anti-SIRPα antibody used in the present invention.

In addition, an antitumor effect of the pharmaceutical composition and therapeutic method of the present invention can be confirmed, in a clinical study, with Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

The foregoing methods can provide the confirmation of superiority in terms of the antitumor effect of the pharmaceutical composition and therapeutic method of the present invention to existing pharmaceutical compositions and therapeutic methods for cancer therapy.

The pharmaceutical composition and therapeutic method of the present invention can retard cancer cell growth, suppress its proliferation, and further can disrupt cancer cells. These effects can allow cancer patients to be free from symptoms caused by cancer or achieve improvement in QOL of a cancer patients, and attain a therapeutic effect by sustaining the lives of the cancer patients. Even if the pharmaceutical composition and therapeutic method of the present invention do not accomplish killing cancer cells, they can achieve higher QOL of cancer patients while achieving longer-term survival, by inhibiting or controlling the growth of cancer cells.

The pharmaceutical composition of the present invention can be expected to exert a therapeutic effect by application as systemic therapy to patients, and additionally, by local application to cancer tissues.

The pharmaceutical composition of the present invention may be administered with containing at least one pharmaceutically suitable ingredient. The pharmaceutically suitable ingredient can be suitably selected and applied from formulation additives or the like that are generally used in the art, in view of the dosage, administration concentration or the like of the antibody-drug conjugate and the anti-SIRPα antibody used in the present invention. For example, the antibody-drug conjugate used in the present invention may be administered as a pharmaceutical composition containing a buffer such as a histidine buffer, an excipient such as sucrose or trehalose, and a surfactant such as polysorbate 80 or 20. The pharmaceutical composition containing the antibody-drug conjugate used in the present invention can preferably be used as an injection, can more preferably be used as an aqueous injection or a lyophilized injection, and can even more preferably be used as a lyophilized injection.

In the case that the pharmaceutical composition containing the antibody-drug conjugate used in the present invention is an aqueous injection, it can preferably be diluted with a suitable diluent and then given as an intravenous infusion. For the diluent, a dextrose solution, physiological saline, and the like, can be exemplified, and a dextrose solution can be exemplified preferably, and a 5% dextrose solution can be exemplified more preferably.

In the case that the pharmaceutical composition containing the antibody-drug conjugate used in the present invention is a lyophilized injection, it can preferably be dissolved in water for injection, and subsequently a required amount can be diluted with a suitable diluent and then given as an intravenous infusion. For the diluent, a dextrose solution, physiological saline, and the like, can be exemplified, and a dextrose solution can be exemplified preferably, and a 5% dextrose solution can be exemplified more preferably.

Examples of the administration route which may be used to administer the pharmaceutical composition of present invention include intravenous, intradermal, subcutaneous, intramuscular, and intraperitoneal routes; and preferably include an intravenous route.

The antibody-drug conjugate used in the present invention can be administered to a human once with intervals of 1 to 180 days, and can preferably be administered once a week, once every 2 weeks, once every 3 weeks, or once every 4 weeks, and can even more preferably be administered, once every 3 weeks. Also, the antibody-drug conjugate used in the present invention can be administered at a dose of about 0.001 to 100 mg/kg, and can preferably be administered at a dose of 0.8 to 12.4 mg/kg. The anti-SIRPα antibody according to the present invention can be administered to a human once with intervals of 1 to 180 days, and can preferably be administered once a week, once every 2 weeks, once every 3 weeks, or once every 4 weeks. Also, the anti-SIRPα antibody according to the present invention can be administered at a dose of about 0.001 to 100 mg/kg per dose.

The pharmaceutical composition and therapeutic method of the present invention may further contain a cancer therapeutic agent other than the antibody-drug conjugate and the anti-SIRPα antibody according to the present invention. The pharmaceutical composition and therapeutic method of the present invention can also be administered in combination with another cancer therapeutic agent, thereby enhancing an antitumor effect. Another cancer therapeutic agent to be used for such purpose may be administered to a subject simultaneously, separately, or sequentially with the pharmaceutical composition of the present invention, or may be administered with varying each dosage interval. Such cancer therapeutic agents are not limited as long as they are agents having antitumor activity, and can be exemplified by at least one selected from the group consisting of irinotecan (CPT-11), cisplatin, carboplatin, oxaliplatin, fluorouracil (5-FU), gemcitabine, capecitabine, doxorubicin, epirubicin, cyclophosphamide, mitomycin C, tegafur-gimeracil-oteracil combination, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation, and lapatinib.

In the pharmaceutical composition and therapeutic method of the present invention, an immune checkpoint inhibitor and an antibody drug specifically binding to a cancer antigen and having ADCC and/or ADCP activity may be further contained as the cancer therapeutic agent to be used in combination, other than the antibody-drug conjugate and the anti-SIRPα antibody according to the present invention. As the immune checkpoint inhibitor, an inhibitor of binding between PD-1 and its ligand, PD-L1, or a CTLA4 inhibitor is exemplified. Specific examples thereof include an anti-PD-1 antibody (nivolumab, pembrolizumab, cemiplimab, spartalizumab, PDR-001, or BI 754091), an anti-PD-L1 antibody (atezolizumab, avelumab, or durvalumab), and an anti-CTLA4 antibody (ipilimumab or tremelimumab). Examples of the antibody drug specifically binding to a cancer antigen and having ADCC activity and/or ADCP activity include an anti-CD20 antibody (rituximab), an anti-HER2 antibody (trastuzumab or pertuzumab), an anti-EGFR antibody (cetuximab), and an anti-CD52 antibody (alemutuzumab).

ADCC refers to a cell-mediated reaction in which non-specific cytotoxic cells (for example, NK cells, neutrophils and macrophages) expressing an Fcy receptor recognize an antibody bound to a target cell, and then, causes lysis of the target cell. In NK cells, which are primary cells responsible for ADCC, FcyRIIC and FcyRIIIA are expressed. In monocytes, FcyRI, FcyRIIA, FcyRIIC and FcyRIIIA are expressed. On the other hand, ADCP refers to a cell-mediated reaction, in which phagocytes (for example, macrophages, neutrophils) expressing an Fc receptor recognizes an antibody bound to a target cell, and then, engulfs the target cell within the cells. In the monocytes, which are primary cells responsible for ADCP, FcyRI, FcyRIIA, FcyRIIC and FcyRIIIA are expressed.

The pharmaceutical composition and therapeutic method of the present invention can also be used in combination with radiotherapy. For example, a cancer patient receives radiotherapy before and/or after or simultaneously with receiving a therapy with the pharmaceutical composition of the present invention.

The pharmaceutical composition and therapeutic method of the present invention can also be used as an adjuvant chemotherapy in combination with surgical operation. The pharmaceutical composition of the present invention may be administered for the purpose of diminishing the size of tumor before surgical operation (referred to as pre-operative adjuvant chemotherapy or neoadjuvant therapy), or may be administered after surgical operation for the purpose of preventing the recurrence of tumor (referred to as post-operative adjuvant chemotherapy or adjuvant therapy).

### Examples

The present invention is specifically described in view of the examples shown below. However, the present invention is not limited to these. Further, it is by no means to be interpreted in a limited way.

### Production Example 1: Preparation of anti-human SIRPα antibody (clone: hD13_H1L3)

Anti-human SIRPα antibody hD13_H1L3 was prepared by a method similar to that described in International Publication No. WO 2020/013170. The amino acid sequences of a heavy chain and light chain of hD13_H1L3 antibody are shown in SEQ ID NOs: 1 and 4.

### Production Example 2: Preparation of anti-mouse SIRPα antibody (clone: 5C12)

Anti-mouse SIRPα antibody 5C12 was established by the following method. For immunization, WKY/Izm or Wister rat female (Japan SLC, Inc.) was used. A mouse SIRPα protein (manufactured by Sino Biological Inc.) and Freund's Complete Adjuvant (manufactured by Wako Pure Chemical Industries, Ltd.) were mixed and subcutaneously administered to the tail root of the rat. The lymph node of the rat was taken and used for production of a hybridoma.

The lymph node cells and mouse myeloma SP2/0-ag14 cells (ATCC: CRL-1581) were fused in accordance with a polyethylene glycol method. The fused cells were suspended in HAT selective medium and cultured in accordance with the limiting dilution method. Hybridoma colonies emerged were collected. In this manner, monoclonal hybridomas were prepared. The hybridoma colonies collected were cultured. Using the resultant hybridoma culture supernatant, an antibody-producing hybridoma was screened based on binding activity to a mouse SIRPα protein as an index. In this manner, clone: 5C12 was screened.

For amplifying cDNA encoding a variable region of 5C12, total RNA was prepared from a 5C12-producing hybridoma by use of TRIzol Reagent (Ambion, Inc.). Based on the total RNA, cDNA encoding heavy chain and light chain variable regions was amplified by use of SMARTer RACE 5'/3' Kit (Clontech, Inc.). The cDNA encoding heavy chain and light chain variable regions amplified by 5'-RACE PCR was cloned to a plasmid. Subsequently, the nucleotide sequence of the cDNA encoding heavy chain and light chain variable regions was analyzed. The amino acid sequences of 5C12 heavy chain and light chain are shown in SEQ ID NOs: 12 and 13. The homology between human SIRPα and mouse SIRPα is as low as 60%. It is reported that humans have 10 types of variants in IgV domain of SIRPα, which is an interaction site with CD47; and mice have at least 4 types of variants due to difference in genetic background. Because of this, it is presumably difficult to obtain a functional antibody having cross-reactivity to the orthologue of a human and a mouse, at which SIRPα-CD47 interaction can be inhibited. Actually, functional antibodies having a cross-reactivity to a mouse have not yet been found in a rat immunized with a human antigen.

A SIRPα molecule is present in myeloid cells such as macrophages and dendritic cells. In the case of a drug directly producing an anti-cancer effect on cancer, generally, the drug can be evaluated by use of a xenograft model prepared by transplanting a human cancer cell line to an immunodeficient mouse. However, in order to evaluate an antitumor effect of a target molecule like SIRPα expressed in host immune cells, any one of the following methods must be employed: (1) using a model, which is prepared by transplanting, to an immunocompetent mouse, a cancer cell line of a mouse having a compatible genetic background, and a surrogate antibody having a cross-reactivity to mouse SIRPα; (2) using a model, which is prepared by introducing a human SIRPα gene into an immunodeficient mouse and transplanting a human cancer cell line thereto, and an anti-human SIRPα antibody; (3) using a mouse model, which is prepared by transplanting a mouse cancer cell line having a human CD47 gene expressed therein to an immunocompetent mouse having an SIRPα target gene introduced therein, and an anti-human SIRPα antibody. Of them, the method (2) has a problem in that since a T cell defective immunodeficient mouse is used, effect on an acquired immune system (immune response mainly by T cells) when SIRPα is inhibited, cannot be evaluated; and the method (3) has a problem in that it takes long time for preparation and an effect of e.g. control of SIRPα gene expression level on an antitumor effect is not easily estimated. On the other hand, in the case of the method (1) using a surrogate antibody, evaluation can be carried out in a general mouse model; in addition, it has been reported that a combined effect is obtained when it is used in combination with an anticancer antibody and an immune checkpoint antibody in clones such as MY-1 and P84 (International Publication No. WO 2017/178653, or Yanagita et al., JCI Insight, 2017 (2)1, 1-15). From this, in this experiment, 5C12 clone was obtained as an anti-mouse SIRPα antibody and an antitumor effect was evaluated. 5C12 has a high binding affinity to a mouse SIRPα antigen. It is confirmed that the binding affinity is at the same level as that of an anti-human SIRPα antibody hD13_H1L3 to a human SIRPα antigen. Note that, a plurality of PD-1 antibodies already marketed were evaluated by use of a surrogate antibody in non-clinical pharmacology studies, and thereafter, the effect of the antibodies were clinically confirmed. From the foregoing, the immunological evaluation and evaluation results of antitumor studies using an anti-SIRPα surrogate antibody can be extrapolated to results in humans.

Example 1: ADCP activity of each of Sacituzumab govitecan (SG) and Polatuzumab vedotin (PV), and an anti-human SIRPα antibody to cancer cell line.

### 1-1 Preparation of target cells

CD47- and TROP2-positive human gastric cancer cell line AGS cells, and CD47- and CD79b-positive human Burkitt's lymphoma Ramos cells were collected, washed twice with PBS and resuspended in PBS. The number of living cells were counted in accordance with the trypan blue staining. A CellTrace Violet (CTV/Thermo Fisher Scientific) solution was added in a volume of 1 µL per 1 × 10⁶ cells/mL. The mixture was allowed to stand still at room temperature for 10 minutes. 10 mL of 10% FBS containing RPMI1640 culture medium (R10) was added and centrifugation was performed at room temperature at 270 × g for 3 minutes. Then, 10 ml of R10 was added and washing was carried out once. Resuspension was carried out so as to be 1 × 10⁶ cells/mL and resuspended cells were used as target cells.

### 1-2 Preparation of effector cells

To SepMate-50 tubes (STEMCELL), Ficoll-Paque PLUS (GE Healthcare) was dispensed in an amount of 15 mL/tube. The whole blood diluted twice with 2% FBS containing PBS was overlaid in an amount of 33.3 mL/tube. Centrifugation was carried out at room temperature at 1200 × g for 10 minutes. The supernatant containing PBMC was collected in fresh 50 mL tubes by decantation and 10 mL of 2% FBS containing PBS was added. Centrifugation was carried out at room temperature at 300 × g for 5 minutes. After the supernatant was removed, 30 mL of 2% FBS containing PBS was added. Centrifugation was carried out at room temperature at 300 × g for 5 minutes to perform washing twice. Suspending with 1 mL of Robosep buffer was carried out, and then, the number of PBMC living cells was counted in accordance with the trypan blue pigment exclusion test. Preparation was carried out with RoboSep buffer so as to be 5 × 10⁷ cells/mL and EasySep human Monocyte enrichment cocktail provided in EasySep human monocyte Enrichment Kit Without CD16 Depletion (STEMCELL) was added in an amount of 50 µL per PBMC cells (5 × 10⁷ cells). After a reaction at 4°C for 10 minutes, EasySep Magnetic Particles were added in an amount of 50 µL per mL per PBMC cells (5 × 10⁷ cells). After a reaction at 4°C for 5 minutes, RoboSep buffer was added so as to be 2.5 mL. Setting was carried out on EasySep Magnet. Two minutes and 30 seconds later, the supernatant was collected. Centrifugation was carried out at 300 x g for 5 minutes, and then, monocyte fractions were collected. R10 containing 20 ng/mL M-CSF (PEPROTEC) was added. Seeding was carried out in a floating 225 cm² flask. Culture was carried out under the conditions of 37°C and 5% CO₂ for 7 days. On Day 7 after initiation of culture, the culture supernatant was removed and the medium was exchanged with fresh R10 containing 20 ng/mL M-CSF. On Day 11 after initiation of culture, the culture supernatant was removed, R10 containing 20 ng/mL IL-10 (PEPROTEC) and 20 ng/mL M-CSF was added. Culture was carried out for further two days. Thirteen days later, TrypLE Express (Life Technology) (7 mL) was added to macrophages induced by differentiation. A reaction was carried out at 37°C for 10 minutes, the macrophages were removed. R10 (23 mL) was added and collection was carried out. Washing was carried out twice with PBS and resuspension was carried out with PBS so as to be 1 × 10⁶ cells/mL. As a standard solution, 1 µL/10⁶ cells/mL CellTrace Far Red solution (ThermoFisher) was added. The mixture was allowed to stand at room temperature for 10 minutes. R10 (10 ml) was added and centrifugation was performed at room temperature at 300 × g for 5 minutes. R10 (10 mL) was added and washing was carried out further once. Resuspension was carried out so as to be 1 × 10⁶ cells/mL and the resuspended cells were used as effector cells.

### 1-3 Evaluation of ADCP activity

To a 96-well (U-bottom) microplate, SG diluted with R10 so as to be a final concentration of 1 µg/mL, PV diluted with R10 so as to be a final concentration of 0.2 µg/mL, a humanized anti-SIRPα antibody (clone: hD13_H1L3) diluted with R10 so as to be a final concentration of 10 µg/mL, an antibody-drug conjugate in combination with a humanized anti-SIRPα antibody, or each of different control groups diluted to be the same final concentrations, was added in an amount of 100 µL/well. To the wells, effector cells prepared in step 1-2 and target cells prepared in step 1-1 were added sequentially in an amount of 50 µL/well. The cells were allowed to stand under the conditions of 37°C and 5% CO₂ for 4 hours. After centrifugation at room temperature at 510 × g for 3 minutes, the supernatant was removed. Washing was carried out with FACS buffer (200 µL/well). Thereafter, 1 × BD Stabilizing Fixative (Becton, Dickison) was added in an amount of 50 µL/well to fix cells. The fixed cells were analyzed by flow cytometry (BD LSRFortessa X-20/BD Biosciences). CTV-positive cells were defined as cancer cells and Far Red positive cells were defined as macrophages. Furthermore, the CTV positive and Far Red positive cells were defined as cancer cells engulfed by macrophages. The ADCP activity (%) was calculated in accordance with the following formula. ADCP (%) = (CTV positive and Far Red positive cell count/CTV positive cell count) × 100

The obtained results were analyzed by GraphPad Prism 9.1.0 (GraphPad Software) and statistical analysis was performed by SAS System Release 9.2 (SAS Institute).

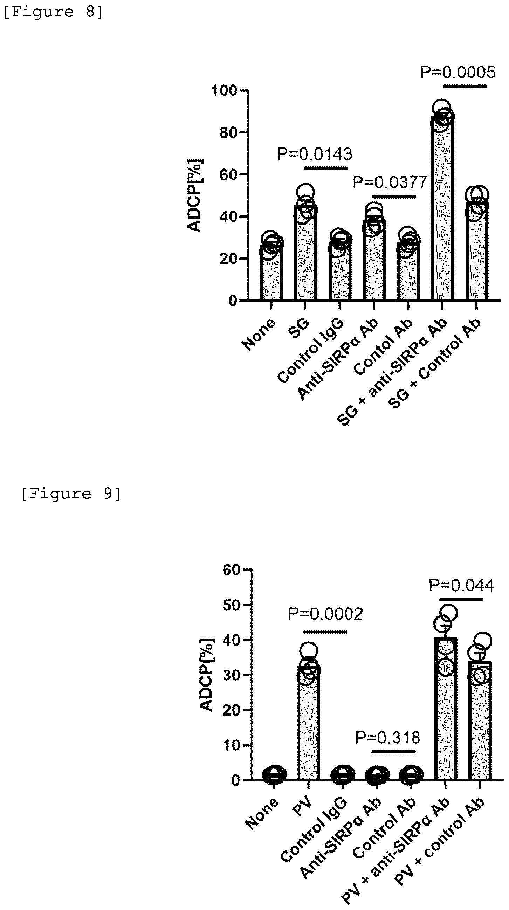

As shown in Figure 8, SG exhibited ADCP activity to CD47 and TROP2-positive human gastric cancer cell line AGS cells. Further, the ADCP activity was increased by combination use with humanized anti-SIRPα antibody hD13_H1L3. Similarly, as shown in Figure 9, PV exhibited ADCP activity to CD47 and CD79b-positive human Burkitt's lymphoma Ramos cells and the ADCP activity was increased by combination use with humanized anti-SIRPα antibody hD13_H1L3. Statistical analysis was performed by Paired t-test and P value was expressed to four decimal places. The results satisfying P < 0.05 (two-sided test) were determined as being significant.

### Example 2: ADCP activity of Trastuzumab emtansine (T-DM1) and anti-human SIRPα antibody to cancer cell line

### 2-1 Preparation of target cells

CD47 and HER2-positive human breast cancer cell line SK-BR-3 cells were collected, washed once with PBS and resuspended in 5 mL of PBS. The number of living cells was counted in accordance with the trypan blue staining. A CellTrace Far Red solution was added individually in a volume of 1 µL per 1 × 10⁶ cells/mL. The mixture was allowed to stand at room temperature for 10 minutes. To this, 20 mL of R10 was added and left to stand at room temperature for 5 minutes. Thereafter, centrifugation was carried out and 10 mL of R10 was added. After washing was carried out once, resuspension was carried out so as to be 1 × 10⁶ cells/mL. The resuspended cells were used as target cells.

### 2-2 Preparation of effector cells

To SepMate-50 tubes, Ficoll-Paque PLUS was dispensed in an amount of 15 mL/tube. The whole blood diluted twice with 2% FBS containing PBS was overlaid in an amount of 34 mL/tube. Centrifugation was carried out at room temperature at 1200 × g for 10 minutes. The supernatant containing PBMC was collected in fresh 50 mL tubes by decantation and 10 mL of 2% FBS containing PBS was added. Centrifugation was carried out at room temperature at 300 × g for 8 minutes. After the supernatant was removed, 25 mL of 2% FBS containing PBS was added. Centrifugation was carried out at room temperature at 300 x g for 5 or for 8 minutes to perform washing twice. After suspending with 1 mL of Robosep buffer, the number of PBMC living cells was counted in accordance with the trypan blue pigment exclusion test. Preparation was carried out with RoboSep buffer so as to be 5 × 10⁷ cells/mL and EasySep human Monocyte enrichment cocktail provided in Easysep Human monocyte Enrichment Kit Without CD16 Depletion was added in an amount of 50 µL per the PBMC cells (5 × 10⁷ cells). After a reaction at 4°C for 10 minutes, EasySep Magnetic Particles were added in an amount of 50 µL per the PBMC cells (5 × 10⁷ cells). After a reaction at 4°C for 5 minutes, RoboSep buffer was added so as to be approximately 2.5 mL. Setting was carried out on EasySep Magnet. Two minutes and 30 seconds later, the supernatant was collected. Centrifugation was carried out at 1200 rpm for 5 minutes, and then, monocyte fractions were collected. R10 containing 20 ng/mL M-CSF was added. Seeding was carried out in a floating 225 cm² flask. Culture was carried out under the conditions of 37°C and 5% CO₂ for 7 days. On Day 7 of initiation of culture, the culture supernatant was removed and the medium was exchanged with fresh R10 containing 20 ng/mL M-CSF. On Day 11 of initiation of culture, the culture supernatant was removed and R10 containing 20 ng/mL IL-10 and 20 ng/mL M-CSF was added and culture was carried out for further 2 days. On Day 13, 3 mL of TrypLE Express was added to macrophages induced by differentiation. A reaction was carried out at 37°C for 15 minutes, and the macrophages were removed. R10 (10 mL) was added and collection was carried out. Washing was carried out twice with PBS and resuspension was carried out with PBS so as to be 1 × 10⁶ cells/mL. As a standard solution, a 1 µL/10⁶ cells/mL CellTrace CFSE solution (ThermoFisher) was added. The mixture was allowed to stand at room temperature for 10 minutes. 10% of R10 (20 ml) was added, and allowed to stand at room temperature for 5 minutes. Centrifugation was carried out at 1200 rpm for 5 minutes. Washing was carried out with 10 mL of R10 once. Resuspension was carried out so as to be 1 × 10⁶ cells/mL. The cells resuspended were used as effector cells.

### 2-3 Evaluation of ADCP activity

To a 96-well (U-bottom) microplate, a humanized anti-SIRPα antibody (clone: hD13_H1L3) diluted with R10 or a control antibody was added in an amount of 50 µL/well. Next, the target cells prepared by the method of step 2-1 were added in an amount of 50 µL/well. Subsequently, the effector cells prepared in step 2-2 were added in an amount of 50 µL/well. Finally, T-DM1 or control ADC was added in an amount of 50 µL/well. The cells were allowed to stand under the conditions of 37°C and 5% CO₂ for 4 hours. After centrifugation at room temperature at 1500 rpm for 2 to 3 minutes, the supernatant was removed. Washing was carried out with 2% FBS containing PBS (150 µL/well). Thereafter, 50 µL/well 1 × BD Stabilizing Fixative was added to fix cells. The fixed cells were analyzed by flow cytometry (Attune NxT/Thermo Fisher). Far Red positive cells were defined as cancer cells and CFSE positive cells were defined as macrophages. Further, the Far Red positive and CFSE positive cells were defined as cancel cells engulfed by macrophages. The ADCP activity (%) was calculated in accordance with the following formula: ADCP (%) = (Far Red positive and CFSE positive cell counts/Far Red positive cell count) × 100

The obtained results were analyzed by GraphPad Prism 9.1.0 (GraphPad Software) and statistical analysis was performed by SAS System Release 9.2 (SAS Institute).

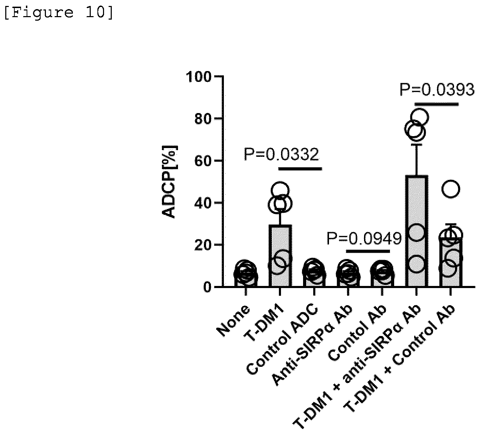

As shown in Figure 10, T-DM1 exhibited ADCP activity to human breast cancer cell line SK-BR-3 cells. Further, the ADCP activity was increased by combination use with humanized anti-SIRPα antibody hD13_H1L3. Statistical analysis was performed by Paired t-test and P value was expressed to four decimal places. The results satisfying P < 0.05 (two-sided test) were determined as being significant.

### Example 3: Antitumor study (1)

6-week-old female C57BL/6J mice (CHARLES RIVER LABORATORIES JAPAN, INC.) were subjected to experiments.

Measurement and calculation formula: the major axis and minor axis of tumors were measured twice a week with an electronic digital caliper (Mitutoyo Corporation), and the tumor volumes (mm³) were calculated. The calculation formula is shown below: Tumor volume (mm3) = 0.5 × Major axis (mm) × [Minor axis (mm) 2

To mouse colorectal cancer cell line MC38 provided from the National Cancer Institute (US, Maryland), a human TROP2 gene was introduced by use of a retroviral vector to obtain MC38-hTROP2 cells, which were put in use. The cells express a human TROP2 protein on the cell membrane. MC38-hTROP2 cells were suspended in saline and 0.5 × 10⁶ cells were transplanted subcutaneously to the right-side abdomen of each of C57BL/6J mice (Day 0). Four days later, the mice were separated into groups at random based on the tumor volumes (Day 4). To a combination group to receive antibody-drug conjugate and anti-SIRPα antibody, Sacituzumab govitecan (SG) diluted with a 10 mM Acetate Buffer and 5% Sorbitol, pH5.5 (ABS) was administered at a dose of 3 mg/kg once in total, on Day 4 via the tail vein. Anti-SIRPα antibody (clone: 5C12) diluted with saline was then intraperitoneally administered at a dose of 10 mg/kg twice in total, on Days 5 and 8. Other than the combination group, a single administration group receiving SG or anti-SIRPα antibody and a control (Vehicle) group receiving ABS and saline were set. The number of mice per group was 8. Measurement of the tumor volume was continued until Day 14.

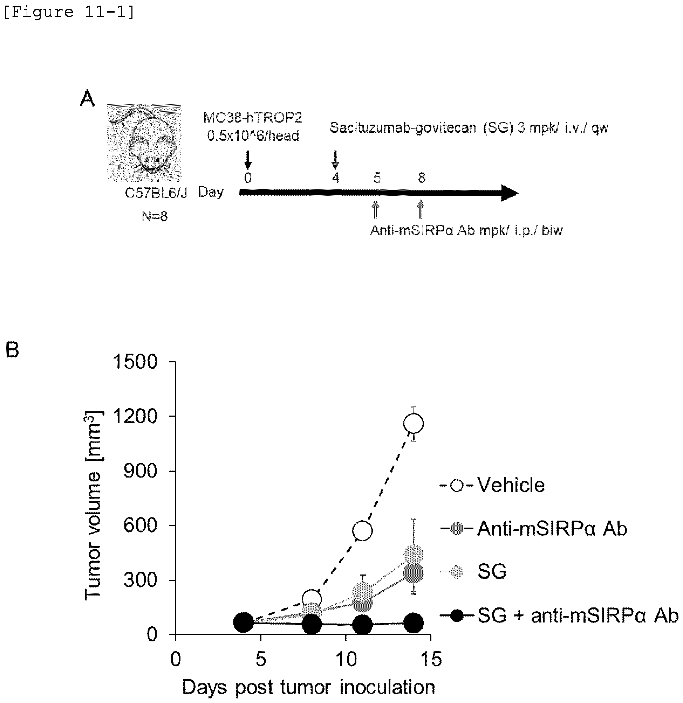

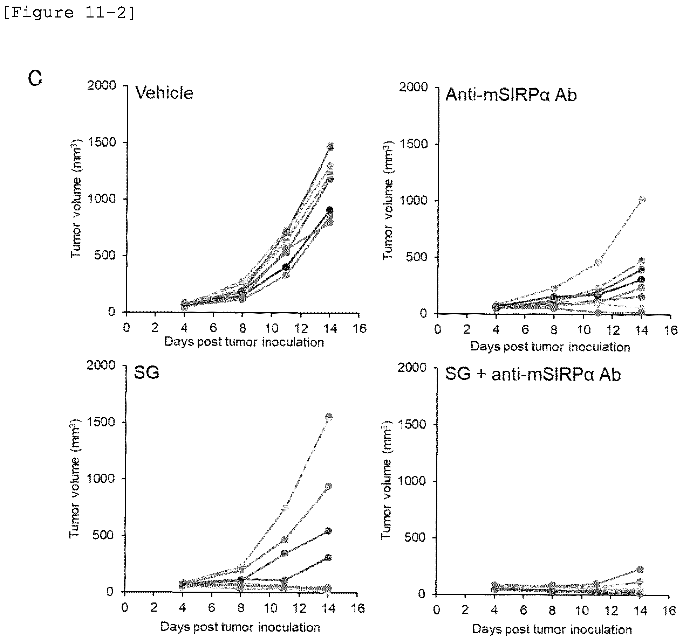

The results are shown in Figure 11. Figure 11A shows the overview of an antitumor study. Figures 11B and C show an average and individual tumor growth curve in individual administration groups, respectively. The longitudinal axis represents tumor volume (mm³) and the abscissa axis represents the number of days after tumor transplantation. On Day 14, the combination group receiving SG and an anti-SIRPα antibody showed the strongest antitumor effect.

### Example 4: Antitumor study (2)

6-week-old female BALB/c mice (BALB/c AnNCrlCrlj) (CHARLES RIVER LABORATORIES JAPAN, INC.) were subjected to experiments.

Measurement and calculation formula: the major axis and minor axis of tumors were measured twice a week with an electronic digital caliper (Mitutoyo Corporation), and the tumor volumes (mm³) were calculated. The calculation formula is shown below: Tumor volume (mm3) = 0.5 × Major axis (mm) × [Minor axis (mm) ]2

To mouse colorectal cancer cell line CT26.WT (CRL2638) purchased from the American Type Culture Collection, a human HER2 gene was introduced by use of a lentivirus vector to prepare CT26.WT-hHER2 cells, which were put in use. The cells express a human HER2 protein on the cell membrane. CT26.WT-hHER2 cells were suspended in PBS (-) and 3.0 × 10⁶ cells were transplanted subcutaneously to the right-side abdomen of each of BALB/c mice (Day -6). Six days later, the mice were separated into groups at random based on the tumor volumes (Day 0). T-DM1 was diluted with saline and administered at a dose of 10 mg/kg twice in total, on Days 0 and 7 via the tail vein. Anti-SIRPα antibody (clone: 5C12) was diluted with saline and intraperitoneally administered at a dose of 10 mg/kg four times in total on Days 0, 3, 7, and 10. Furthermore, single administration group receiving T-DM1 or anti-SIRPα antibody and a control (Vehicle) group receiving saline were set. The number of mice per group was 6. Measurement of the tumor volume was continued until Day 14. Comparison among each of the single administration groups and the combination groups was carried out in accordance with the Dunnett's multiple comparison. P value was expressed to four decimal places and the results satisfying P < 0.05 (two-sided test) were determined as being significant.

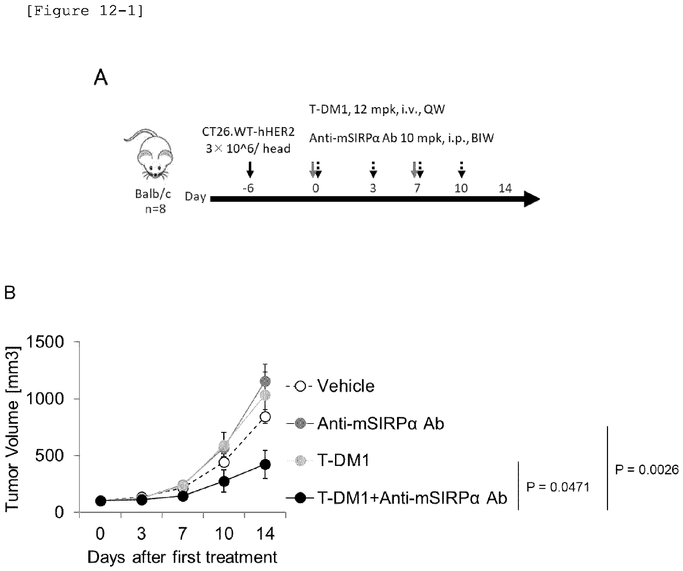

The results are shown in Figure 12. Figure 12A shows the overview of an antitumor study. Figures 12B and C show an average and individual tumor growth curve in individual administration groups, respectively. The longitudinal axis represents tumor volume (mm³) and the abscissa axis represents the number of days after initial administration of a drug. On Day 14, a combination group receiving T-DM1 and anti-SIRPα antibody showed a significantly excellent antitumor effect compared to each of the single administration groups.

### Example 5: Antitumor test (3)

CT26.WT-hNECTIN-4 cells or MC38-hNECTIN-4 cells are used, produced by introducing a human NECTIN-4 gene into mouse colorectal cancer cell line CT26.WT or mouse colorectal cancer cell line MC38 by use of a lentiviral vector. Sometimes a chimera NECTIN-4 (mNECTIN-4_I75A_N90S) of a mouse and a human having an epitope to which Enfortumab vedotin can be bound may be expressed in place of hNECTIN-4. These cells are subcutaneously transplanted in BALB/c or C57BL/6J mice, and then Enfortumab vedotin and an anti-SIRPα antibody (clone: 5C12) are administered and a combination of them is evaluated.

From the above experimental results, it was found that the antibody-drug conjugate according to the present invention administered in combination with the anti-SIRPα antibody exerts a more excellent antitumor effect than each of the effects obtained by single administration of these.

### Free Text of Sequence Listing

SEQ ID NO: 1 - Amino acid sequence of hH1 heavy chain of the humanized anti-SIRPα antibody hD13
SEQ ID NO: 2 - Amino acid sequence of hH2 heavy chain of the humanized anti-SIRPα antibody hD13
SEQ ID NO: 3 - Amino acid sequence of hL2 light chain of the humanized anti-SIRPα antibody hD13
SEQ ID NO: 4 - Amino acid sequence of hL3 light chain of the humanized anti-SIRPα antibody hD13
SEQ ID NO: 5 - Amino acid sequence of hL4 light chain of the humanized anti-SIRPα antibody hD13
SEQ ID NO: 6 - Amino acid sequence of the chimera SIRPα antibody cD13 CDR-H1
SEQ ID NO: 7 - Amino acid sequence of the chimera SIRPα antibody cD13 CDR-H2
SEQ ID NO: 8 - Amino acid sequence of the chimera SIRPα antibody cD13 CDR-H3
SEQ ID NO: 9 - Amino acid sequence of the chimera SIRPα antibody cD13 CDR-L1
SEQ ID NO: 10- Amino acid sequence of the chimera SIRPα antibody cD13 CDR-L2
SEQ ID NO: 11 - Amino acid sequence of the chimera SIRPα antibody cD13 CDR-L3
SEQ ID NO: 12 - Amino acid sequence of a heavy chain of the 5C12 anti-mouse SIRPα antibody
SEQ ID NO: 13 - Amino acid sequence of a light chain of the 5C12 anti-mouse SIRPα antibody

## Claims

1. A pharmaceutical composition, wherein an antibody-drug conjugate and an anti-SIRPα antibody are administered in combination, and
the antibody-drug conjugate is any one antibody-drug conjugate selected from the group consisting of Sacituzumab govitecan, Polatuzumab vedotin, and Trastuzumab emtansine.

2. The pharmaceutical composition according to claim 1, wherein the antibody-drug conjugate is Sacituzumab govitecan.

3. The pharmaceutical composition according to claim 1, wherein the antibody-drug conjugate is Polatuzumab vedotin.

4. The pharmaceutical composition according to claim 1, wherein the antibody-drug conjugate is Trastuzumab emtansine.

5. The pharmaceutical composition according to any one of claims 1 to 4, wherein the anti-SIRPα antibody is any one of the following antibodies (1) to (5):
(1) an antibody comprising a heavy chain consisting of an amino acid sequence of amino acid number 20 or 466 in SEQ ID NO: 1 and
a light chain consisting of an amino acid sequence of amino acid number 21 or 234 in SEQ ID NO: 4;
(2) an antibody comprising a heavy chain consisting of an amino acid sequence of amino acid number 20 or 466 in SEQ ID NO: 1 and a light chain consisting of an amino acid sequence of amino acid number 21 or 234 shown in SEQ ID NO: 5;
(3) an antibody comprising a heavy chain consisting of an amino acid sequence of amino acid number 20 or 466 in SEQ ID NO: 2 and a light chain consisting of an amino acid sequence of amino acid number 21 or 234 shown in SEQ ID NO: 3;
(4) an antibody comprising a heavy chain consisting of an amino acid sequence of amino acid number 20 or 466 in SEQ ID NO: 2 and a light chain consisting of an amino acid sequence of amino acid number 21 or 234 shown in SEQ ID NO: 4; and
(5) an antibody according to any one of (1) to (4) lacking a lysine residue at the carboxyl terminus of the heavy chain.

6. The pharmaceutical composition according to any one of claims 1 to 5, wherein the antibody-drug conjugate and the anti-SIRPα antibody are contained separately in different preparations as active ingredients and are administered simultaneously or at different times.

7. The pharmaceutical composition according to any one of claims 1 to 6, for use in treatment of at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

8. A therapeutic method comprising administering an antibody-drug conjugate and an anti-SIRPα antibody in combination to a subject in need of treatment, wherein the antibody-drug conjugate is any one antibody-drug conjugate selected from the group consisting of Sacituzumab govitecan, Polatuzumab vedotin, and Trastuzumab emtansine.

9. The therapeutic method according to claim 8, wherein the antibody-drug conjugate is Sacituzumab govitecan.

10. The therapeutic method according to claim 8, wherein the antibody-drug conjugate is Polatuzumab vedotin.

11. The therapeutic method according to claim 8, wherein the antibody-drug conjugate is Trastuzumab emtansine.

12. The therapeutic method according to any one of claims 8 to 11, wherein the anti-SIRPα antibody is any one of the following antibodies (1) to (5):
(1) an antibody comprising a heavy chain consisting of an amino acid sequence consisting of amino acid residues 20 to 466 of SEQ ID NO: 1 and a light chain consisting of an amino acid sequence consisting of amino acid residues 21 to 234 of SEQ ID NO: 4;
(2) an antibody comprising the heavy chain consisting of the amino acid sequence consisting of amino acid residues 20 to 466 of SEQ ID NO: 1 and a light chain consisting of an amino acid sequence consisting of amino acid residues 21 to 234 of SEQ ID NO: 5;
(3) an antibody comprising a heavy chain consisting of an amino acid sequence consisting of amino acid residues 20 to 466 of SEQ ID NO: 2 and a light chain consisting of an amino acid sequence consisting of amino acid residues 21 to 234 of SEQ ID NO: 3;
(4) an antibody comprising the heavy chain consisting of the amino acid sequence consisting of amino acid residues 20 to 466 of SEQ ID NO: 2 and a light chain consisting of an amino acid sequence consisting of amino acid residues 21 to 234 of SEQ ID NO: 4; and
(5) an antibody according to any one of (1) to (4), lacking a lysine residue at the carboxyl terminus of the heavy chain.

13. The therapeutic method according to any one of claims 8 to 12, wherein the antibody-drug conjugate and the anti-SIRPα antibody are separately contained as active components in different formulations, and are administered simultaneously or at different times.

14. The therapeutic method according to any one of claims 8 to 13, wherein the method is for treating at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine cancer, sarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

15. An anti-SIRPα antibody to be administered in combination with an antibody-drug conjugate, wherein the antibody-drug conjugate is any one antibody-drug conjugate selected from the group consisting of Sacituzumab govitecan, Polatuzumab vedotin, and Trastuzumab emtansine.

16. The anti-SIRPα antibody according to claim 15, wherein the antibody-drug conjugate is Sacituzumab govitecan.

17. The anti-SIRPα antibody according to claim 15, wherein the antibody-drug conjugate is Polatuzumab vedotin.

18. The anti-SIRPα antibody according to claim 15, wherein the antibody-drug conjugate is Trastuzumab emtansine.

19. The anti-SIRPα antibody according to any one of claims 15 to 18, wherein the anti-SIRPα antibody is any one of the following antibodies (1) to (5):
(1) an antibody comprising a heavy chain consisting of an amino acid sequence of amino acid number 20 or 466 in SEQ ID NO: 1 and a light chain consisting of an amino acid sequence of amino acid number 21 or 234 shown in SEQ ID NO: 4;
(2) an antibody comprising a heavy chain consisting of an amino acid sequence of amino acid number 20 or 466 in SEQ ID NO: 1 and a light chain consisting of an amino acid sequence of amino acid number 21 or 234 shown in SEQ ID NO: 5;
(3) an antibody comprising a heavy chain consisting of an amino acid sequence of amino acid number 20 or 466 in SEQ ID NO: 2 and a light chain consisting of an amino acid sequence of amino acid number 21 or 234 shown in SEQ ID NO: 3;
(4) an antibody comprising a heavy chain consisting of an amino acid sequence of amino acid number 20 or 466 in SEQ ID NO: 2 and a light chain consisting of an amino acid sequence of amino acid number 21 or 234 shown in SEQ ID NO: 4; and
(5) an antibody according to any one of (1) to (4) lacking a lysine residue at the carboxyl terminus of the heavy chain.

20. The anti-SIRPα antibody according to any one of claims 15 to 19, wherein an antibody-drug conjugate and the anti-SIRPα antibody are contained separately in different preparations as active ingredients and administered simultaneously or at different times.

21. The anti-SIRPα antibody according to any one of claims 15 to 20, for use in treatment of at least one selected from the group consisting of breast cancer, gastric cancer, colorectal cancer, lung cancer, esophageal cancer, salivary gland cancer, esophagogastric junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
